# EUROPEAN PATENT APPLICATION

(11) **EP 4 331 629 A1**
(43) Date of publication of application: **06.03.2024**
(21) Application number: 22795282.7
(22) Date of filing: 04.03.2022
(51) Int. Cl.: A61L 2/10, A61L 9/20

(54) **ULTRAVIOLET LIGHT EMISSION DEVICE, METHOD FOR USING ULTRAVIOLET LIGHT EMISSION DEVICE, AND ULTRAVIOLET LIGHT EMISSION METHOD**

(30) Priority: 30.04.2021 JP 2021077022
(71) Applicant: Ushio Denki Kabushiki Kaisha, Tokyo 100-8150 (JP)
(72) Inventor: FUJINA,Kyosuke, Tokyo 100-8150 (JP)
(74) Representative: Maiwald GmbH
(86) International application number: PCT/JP2022/009419
(87) International publication number: WO 2022/230359

(57) **Abstract**

Provided are an ultraviolet irradiation device that can effectively inactivate pathogens while ensuring safety for a human body, a method for using the irradiation device, and the ultraviolet irradiation method. The ultraviolet irradiation device according to the present invention includes: a light source that emits ultraviolet light having a main emission wavelength belonging to a range from 190 nm to 240 nm; a housing that accommodates the light source; an extraction part that extracts the ultraviolet light emitted from the light source to the outside of the housing; and a diverging optical system that enlarges a light distribution angle of the ultraviolet light passing through the extraction part.

## Description

### TECHNICAL FIELD

The present invention relates to an ultraviolet irradiation device, a method for using the ultraviolet irradiation device, and an ultraviolet irradiation method.

### BACKGROUND ART

It is known that pathogens such as bacteria, fungi, and viruses have an absorption maximum around a wavelength of 260 nm. Therefore, there has been conventionally known a technique of inactivating a pathogen by emitting an object surface or a space where the pathogen is present with ultraviolet light having a high emission spectrum around a wavelength of 254 nm using a low-pressure mercury lamp.

For example, Patent Document 1 describes that a sterilization lamp that emits ultraviolet light is attached to a kitchen or the like to sterilize the kitchen. Further, Patent Document 2 describes an action of sterilization by emitting ultraviolet light to bacteria and viruses floating in a room.

Still further, ultraviolet irradiation devices described in Patent Documents 1 and 2 use ultraviolet light in a wavelength band harmful to a human body. Therefore, measures are taken such as giving directivity to the emitted ultraviolet light so that the ultraviolet light is not directed to the human body.

### PRIOR ART DOCUMENTS

### PATENT DOCUMENTS

Patent Document 1: JP-U-63-187221
Patent Document 2: JP-A-2017-018442

### SUMMARY OF THE INVENTION

### PROBLEMS TO BE SOLVED BY THE INVENTION

However, pathogens such as bacteria, fungi and viruses are present in every part of the space. Pathogens are often contained in airborne droplets released from the mouths or noses of humans and spread through expired air or saliva or by coughing or sneezing, or contained in aerosol. The airborne droplets containing the pathogens adhere to a human body surface (for example, skin and hair) or an object surface with which a person comes into contact (for example, furniture and office equipment). The aerosol containing the pathogens floats and diffuses in the space.

Due to the risk of emitting the harmful ultraviolet light to the human body, the conventional ultraviolet irradiation device emits ultraviolet light only in a direction where people are not present. Therefore, the conventional ultraviolet irradiation device has a limit in inactivation of the pathogens.

The present invention provides an ultraviolet irradiation device that can effectively inactivate pathogens while ensuring safety, a method for using the ultraviolet irradiation device, and an ultraviolet irradiation method.

### MEANS FOR SOLVING THE PROBLEMS

An ultraviolet irradiation device according to the present invention includes:
a light source that emits ultraviolet light having a main emission wavelength belonging to a range from 190 nm to 240 nm;
a housing that accommodates the light source;
an extraction part that extracts the ultraviolet light emitted from the light source to the outside of the housing; and
a diverging optical system that enlarges a light distribution angle of the ultraviolet light passing through the extraction part.

By enlarging the light distribution angle of the ultraviolet light by the diverging optical system, the ultraviolet light can be emitted over a wide range. Therefore, the ultraviolet light can be emitted to every corner of the space where the floating pathogens possibly exist. In addition, the cost effect is excellent from the viewpoint that a region where microorganisms are desired to be inactivated can be covered by a small number of ultraviolet irradiation devices.

The ultraviolet light does not present harmfulness to the human body in all wavelength bands. It has been reported that ultraviolet light having a wavelength of 240 nm or more is harmful to human cells and animal cells, but ultraviolet light having a wavelength band shorter than the wavelength band of the above ultraviolet light has a small penetrating force to human cells and animal cells, and therefore has extremely low harmfulness to humans and animals.

The present inventor has made it possible to emit the ultraviolet light in a direction where humans and animal are present by using ultraviolet light having a wavelength of 190 nm or more and less than 240 nm having extremely low harmfulness. This enables inactivation of the pathogens while the safety is ensured. Herein, "inactivation" refers to a concept that includes killing bacteria or viruses or making infectivity or toxicity of bacteria or viruses lost.

A target product covered by the present invention can provide sterilization and virus inactivation performance intrinsic to the ultraviolet light without causing erythema or keratitis on the skin or eyes of humans or animals. In particular, the target product of the present invention has a characteristic that it can be used in a manned environment unlike the conventional ultraviolet light source. By utilizing this feature and installing the target product of the present invention in the indoor or outdoor manned environment, the entire environment can be emitted by the ultraviolet light, and the viruses or bacteria present in the air or on the surface of the member installed in the environment can be suppressed.

This accords with Goal 3 "Ensure healthy lives and promote well-being for all at all ages" included in the Sustainable Development Goals (SDGs) led by the United Nations, and will greatly contribute to the goal target 3.3 "By 2030, end the epidemics of AIDS, tuberculosis, malaria and neglected tropical diseases and combat hepatitis, water-borne diseases and other communicable diseases".

In a case where the pathogens present on an object surface or in a space are inactivated using the ultraviolet light having a wavelength of 190 nm or more and less than 240 nm, irradiance (light intensity) and an irradiation dose (integrated light quantity) of ultraviolet light to be emitted to the object surface or the space to be a target become factors that determine an effect of inactivation.

For example, American Conference of Governmental Industrial Hygienists (ACGIH) or JIS Z 8812 (Measuring methods of eye-hazardous ultraviolet radiation) specifies that the irradiation dose of ultraviolet light to a human body per day (8 hours) should be equal to or less than a threshold limit value (TLV) dependent on wavelength. As described above, the harmfulness of ultraviolet light having a wavelength of 190 nm or more and less than 240 nm is extremely low, but in order to perform safe operation, it is desirable to limit the irradiance and the irradiation dose of the ultraviolet light emitted per predetermined time so as not to exceed the threshold limit value.

According to intensive research by the present inventors, it has been found that the irradiance of ultraviolet light emitted from the ultraviolet irradiation device is not uniform but exhibits a biased alignment distribution (includes unevenness). Therefore, in setting the operation of the ultraviolet irradiation device in consideration of the TLV, it is necessary to set the upper limit of the irradiation dose (integrated light quantity) of the ultraviolet light in accordance with the region locally receiving a strong light. This causes the irradiation dose of the ultraviolet light in the region locally receiving a weak light to be restricted more than necessary.

According to the present invention, by using the diverging optical system, it is possible to diverge the locally strong light beam to make the alignment distribution of the light intensity uniform. As a result, in setting the operation of the ultraviolet irradiation device in consideration of the TLV, the upper limit of the ultraviolet light irradiation dose is less likely to be restricted. Therefore, by using the diverging optical system, it is possible to ensure safety at a higher level and to be less likely to be restricted by the upper limit of the ultraviolet light irradiation dose.

In order to ensure safety at a higher level, the local maximum irradiance of ultraviolet light (irradiance in a local region to which the most intense light is emitted) may be suppressed by the diverging optical system. For example, the local maximum irradiance of ultraviolet light on a light-emitting surface of the diverging optical system may be suppressed to 3 mW/cm² or less, and further, 1 mW/cm² or less.

The diverging optical system has characteristics that attenuation of light is small and emission efficiency is improved. In addition, the diverging optical system can have an optical design aiming at a desired light distribution angle.

An optical filter that transmits the ultraviolet light having the main emission wavelength belonging to the wavelength band from 190 nm to 240 nm and does not substantially transmit the ultraviolet light having the wavelength band from 240 nm to 280 nm may be provided on the incident side of the diverging optical system. This reliably suppresses the ultraviolet light in the wavelength band that possibly affect the human body from leaking to the outside of the housing, and the safety of the irradiation device with respect to the human body is further improved. Furthermore, even in a case where the light distribution angle is reduced using the optical filter, a large light distribution angle can be obtained by disposing the diverging optical system on the emission side of the optical filter.

A separation distance between the optical filter and the diverging optical system may be 0 mm or more and 3 mm or less. The emission light of the optical filter can be effectively incident on the diverging optical system, and the emission efficiency can be enhanced.

The diverging optical system may include a lens array.

The light source may include at least one excimer lamp,
the lens array includes small lenses whose number may be equal to the number of the excimer lamps, and
the longitudinal direction of the small lens may be along the longitudinal direction of the excimer lamp. This causes the lens array to effectively diverge the emission light from the excimer lamp.

A method for using the ultraviolet irradiation device according to the present invention includes disposing the ultraviolet irradiation device such that at least a part of the emitted ultraviolet light is emitted toward a space, and causing the ultraviolet irradiation device to emit the ultraviolet light.

An ultraviolet irradiation device according to the present invention includes:
a light source that emits ultraviolet light having a main emission wavelength belonging to a range from 190 nm to 240 nm;
a housing that accommodates the light source; and
an extraction part that extracts the ultraviolet light emitted from the light source to the outside of the housing, in which
the housing includes an attachment part configured to attach a diverging optical system that enlarges a light distribution angle of the ultraviolet light passing through the extraction part. This allows the diverging optical system to be retrofitted to the ultraviolet irradiation device.

An ultraviolet irradiation method according to the present invention includes:
emitting ultraviolet light having a main emission wavelength belonging to a range from 190 nm to 240 nm from a light source; and
enlarging a light distribution angle of the ultraviolet light using a diverging optical system.

### EFFECT OF THE INVENTION

With this configuration, it possible to provide the ultraviolet irradiation device that can effectively inactivate the pathogens while the safety is ensured, the method of using the ultraviolet irradiation device, and the ultraviolet irradiation method.

### BRIEF DESCRIPTION OF THE DRAWINGS

Fig. 1 is a perspective view schematically illustrating the appearance of an ultraviolet irradiation device.
Fig. 2 is a perspective view schematically illustrating the appearance of the ultraviolet irradiation device.
Fig. 3 is a perspective view illustrating only a light source and an electrode block extracted from the ultraviolet irradiation device.
Fig. 4 is a cross-sectional view in a plane C1 in Fig. 1.
Fig. 5 is a view for explaining an effect of enlarging a light distribution angle of emitted light by a diverging optical system.
Fig. 6 is an example of an emission spectrum of an excimer lamp in which a luminescent gas contains KrCI.
Fig. 7 is a graph illustrating an example of a transmission spectrum of an optical filter.
Fig. 8 is a schematic view for illustrating an incident angle of ultraviolet light on the optical filter.
Fig. 9 is a schematic cross-sectional view illustrating a first modification of an ultraviolet irradiation device.
Fig. 10 is a schematic cross-sectional view illustrating a second modification of an ultraviolet irradiation device.
Fig. 11 is a schematic cross-sectional view illustrating a second embodiment of an ultraviolet irradiation device.
Fig. 12 is a schematic view of a measurement facility for measuring a light distribution angle of an ultraviolet irradiation device.
Fig. 13 is a schematic cross-sectional view illustrating a diverging optical system used in examples.
Fig. 14 is a graph showing an angular distribution of relative irradiance of the ultraviolet irradiation device.

### MODE FOR CARRYING OUT THE INVENTION

An ultraviolet irradiation device according to an embodiment is described with reference to the drawings. Note that the following drawings are schematically illustrated, the dimensional ratio in the drawings do not necessarily coincide with the actual dimension ratio, and the dimensional ratios do not necessarily coincide between the drawings.

Hereinafter, each of the drawings is described with reference to an XYZ coordinate system as appropriate. In the XYZ coordinate system, a traveling direction of a light beam of emitted ultraviolet light on an optical axis is defined as the +X direction, and a plane orthogonal to the X direction is defined as a YZ plane. In describing directions in the present description, in a case of distinguishing whether the direction is positive or negative, the positive or negative symbol is added, such as the "+X direction" or the "-X direction". In a case where there is no need to distinguish between the positive and negative directions, the direction is simply described as the "X direction". Namely, in the present description, in the case where the direction is simply described as the "X direction", both "+X direction" and "-X direction" are included. The same applies to the Y direction and the Z direction.

### <First embodiment>

### [Outline of ultraviolet irradiation device]

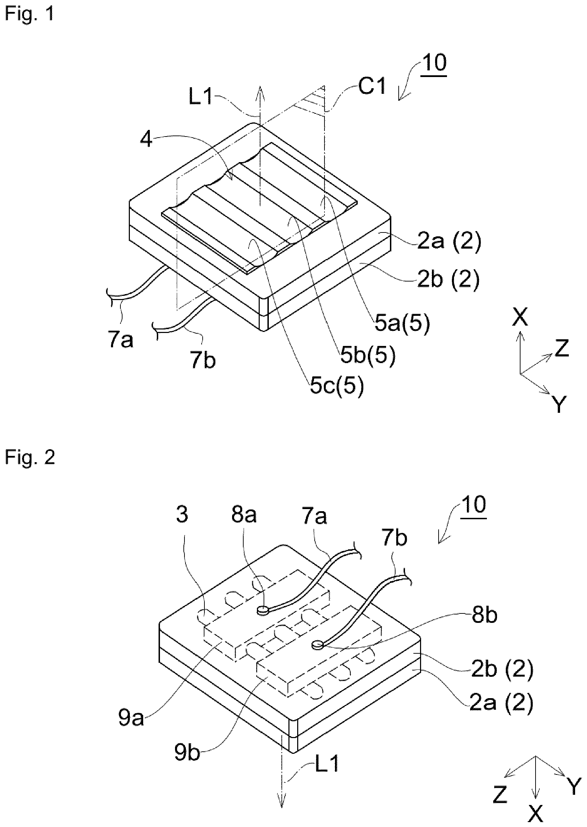

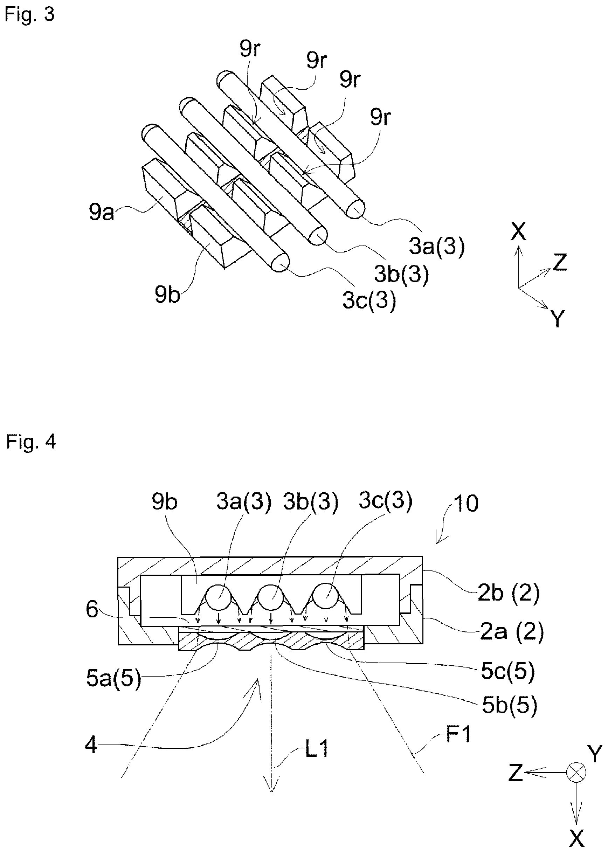

An outline of an embodiment of an ultraviolet irradiation device is described with reference to Figs. 1, 2, and 3. Figs. 1 and 2 are perspective views schematically illustrating the appearance of the ultraviolet irradiation device. Fig. 3 is a perspective view illustrating only a light source and an electrode block extracted from the ultraviolet irradiation device.

An ultraviolet irradiation device 10 of the present embodiment includes excimer lamps 3 that emit ultraviolet light (see Figs. 2 and 3), a housing 2 that accommodates the excimer lamp 3, an extraction part 4 that extracts the ultraviolet light emitted from the excimer lamp 3 to the outside of the housing 2 in the +X direction, a diverging optical system 5 that causes the ultraviolet light to diverge, and an optical filter described later. In Figs. 1 and 2, an arrow L1 indicates an optical axis of ultraviolet light emitted from the excimer lamps 3 and a traveling direction of a light beam on the optical axis.

In the present embodiment, the housing 2 includes a first frame 2a having an opening that functions as the extraction part 4 in the center and a second frame 2b having no opening. The second frame 2b and the first frame 2a are fitted to each other to form an internal space surrounded by the housing 2. In this internal space, the excimer lamps 3 and two electrode blocks (9a, 9b) that supplies power to the excimer lamps 3 are disposed. The frame constituting the housing 2 may include three or more frames.

The two electrode blocks (9a, 9b) are fixed to the inner surface of the second frame 2b (see Fig. 2). Two connection terminals (8a, 8b) are provided on the outer surface of the second frame 2b (see Fig. 2). The two connection terminals (8a, 8b) are respectively electrically connected to the electrode blocks (9a, 9b) with the second frame 2b interposed therebetween. The two connection terminals (8a, 8b) are respectively connected to power feeding lines (7a, 7b) fed with power from an external power supply (not illustrated).

### [Light source]

One embodiment of the light source is described with reference to Fig. 3. The present embodiment is provided with three pieces of the excimer lamps 3 (3a, 3b, 3c) arranged so as to be separated from each other in the Z direction. The two electrode blocks (9a, 9b) are in contact with the outer surface of a light-emitting tube of each of the excimer lamps 3 (3a, 3b, 3c). This allows the excimer lamps 3 to be powered and turned on.

In the present embodiment, the excimer lamp 3 that is used is a KrCI excimer lamp having a light-emitting tube filled with a light-emitting gas containing KrCI. Therefore, the excimer lamp 3 emits ultraviolet light having a wavelength of 190 nm to 240 nm. In particular, the KrCI excimer lamp emits ultraviolet light having a main peak wavelength of around 222 nm.

The excimer lamp 3 is not limited to the KrCI excimer lamp. For example, a KrBr excimer lamp may be used which has a light-emitting tube filled with a light-emitting gas containing KrBr. The KrBr excimer lamp emits ultraviolet light having a main peak wavelength of around 207 nm.

In this description, the "main emission wavelength" indicates, in a case where a wavelength range Z(λ) of ±10 nm with respect to a certain wavelength λ is defined on an emission spectrum, a wavelength λi in a wavelength range Z(Ai) showing integrated intensity of 40% or more with respect to the total integrated intensity in the emission spectrum. For example, in a light source having an extremely narrow half-value width and showing light intensity only at a specific wavelength such as an excimer lamp in which a light-emitting gas containing KrCl, KrBr, and ArF is sealed, a wavelength having the highest relative irradiance (main peak wavelength) may be usually regarded as the main emission wavelength.

Regarding the size of the light-emitting tube of the excimer lamp 3, a length in the tube axis direction (Y direction) is preferably 15 mm or more and 200 mm or less, and an outer diameter is preferably 2 mm or more and 16 mm or less. The number of excimer lamps 3 may be one, two, or four or more.

### [Diverging optical system]

Fig. 4 is a schematic cross-sectional view taken along a plane C1 (plane parallel to the XZ plane) of the ultraviolet irradiation device 10 in Fig. 1. In the ultraviolet irradiation device 10 of the present embodiment, the diverging optical system 5 and an optical filter 6 are disposed in the extraction part 4 that extracts the ultraviolet light to the outside of the housing 2. The state in which the diverging optical system 5 or the optical filter 6 is disposed in the extraction part 4 includes a state in which the diverging optical system 5 or the optical filter 6 is inserted into the opening of the housing 2 constituting the extraction part 4 or a state in which the diverging optical system 5 or the optical filter 6 covers the opening of the housing 2. This allows the ultraviolet light passing through the extraction part 4 to pass through the diverging optical system 5 or the optical filter 6 disposed in the extraction part 4. The diverging optical system 5 or the optical filter 6 may be disposed at a position spaced apart from the outer surface of the first frame 2a by a minute distance (for example, within 30 mm) in the X direction.

The diverging optical system 5 has a function of increasing the light distribution angle of the ultraviolet light passing through the extraction part 4. As can be seen from a light beam flux F1 illustrated in Fig. 4, the light beam flux F1 transmitted through the diverging optical system 5 diverges, and the light distribution angle of the emitted light is enlarged.

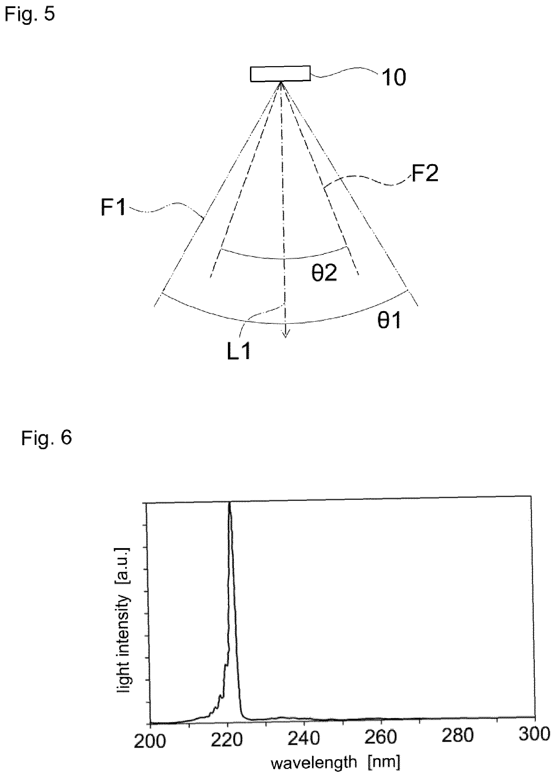

The enlargement of the light distribution angle of the emitted light is described with reference to Fig. 5. In Fig. 5, if the diverging optical system is not included, a light beam flux F2 of the ultraviolet light is emitted from the ultraviolet irradiation device 10 at a light distribution angle θ2 around the optical axis (arrow L1). In a case where the diverging optical system 5 is included, the light beam flux F1 of the ultraviolet light is emitted at a light distribution angle θ1. The light distribution angle θ1 in the case of having the diverging optical system 5 is larger than the light distribution angle θ2 in the case of not having the diverging optical system. The light distribution angle (θ1, θ2) is defined as an angle formed by outermost opposing light beams farthest from the optical axis of the light beam flux (F1, F2) spreading around the optical axis. Note that the light beam flux (F1, F2) spreads around the optical axis as the center and is defined as a flux of light beams having luminance of 1/2 or more with respect to the luminance of the light beam on the optical axis L1 indicating the maximum luminance of the light beam.

By enlarging the light distribution angle of the ultraviolet light by the diverging optical system 5, the ultraviolet irradiation device 10 can emit the ultraviolet light over a wide range. Further, a locally strong light beam is diverged by the diverging optical system 5 to cause the alignment distribution of the light intensity to be uniform.

Returning to Fig. 4, the diverging optical system 5 of the present embodiment includes an optical lens. In the ultraviolet irradiation device using the diverging optical system, because attenuation of light is small, 90% or more of the total light flux is easily maintained. As the optical lens, quartz glass or sapphire glass having high transmittance with respect to the ultraviolet light is preferably used. The optical lens can also be designed such that the light distribution angle belongs to a desired range. As another example of the diverging optical system 5, a mirror may be used for all or a part of the diverging optical system 5.

In the present embodiment, the optical lens constituting the diverging optical system 5 is constituted by a lens array in which a plurality of small lenses (5a, 5b, 5c) is arranged in the Z direction. Each of the small lenses (5a, 5b, 5c) is constituted of a biconcave lens having a cylindrical surface (surface curved in a columnar shape). The longitudinal direction of each of the small lenses (5a, 5b, 5c) (direction in which the cylinder extends) is along the longitudinal direction of the excimer lamp (3a, 3b, 3c). The number of small lenses (5a, 5b, 5c) constituting the lens array is the same as the number of excimer lamps (3a, 3b, 3c). The excimer lamp 3a and the small lens 5a are arranged side by side in the X direction. Similarly, the excimer lamps (3b, 3c) and the small lenses (5b, 5c) are arranged side by side in the X direction. This causes each of the small lenses (5a, 5b, 5c) to effectively diverge the light emitted from each of the excimer lamps (3a, 3b, 3c).

The lens array may be a lens having a configuration in which a plurality of small lenses are arranged in each of the Z direction and the Y direction. In addition, other lens shapes such as a plano-concave lens and a concave meniscus lens may be used as long as the lens has a diverging function. The small lenses constituting the lens array are arranged at an optional pitch. The arrangement pitch of the small lenses is more preferably set to be equal to the arrangement pitch of the excimer lamp.

### [Optical filter]

In the present embodiment, the optical filter 6 is disposed on the incident side of the diverging optical system 5. The optical filter 6 functions as a wavelength selection filter (band pass filter) that transmits ultraviolet light in a specific wavelength band and does not substantially transmit ultraviolet light in a specific wavelength band. The optical filter 6 of the present embodiment transmits the ultraviolet light in a wavelength band of 190 nm or more and less than 240 nm and does not substantially transmit the ultraviolet light in a wavelength band of 240 nm or more and 280 nm or less.

As shown in Fig. 6, for example, in the case of the KrCI excimer lamp, in the spectrum of the emitted ultraviolet light, while the light output is concentrated at or near 222 nm, which is almost the main peak wavelength, there is also a slight light output recognized for the ultraviolet light in the wavelength band of 240 nm or more and 280 nm or less, which may affect the human body. In the present embodiment, by disposing the optical filter 6 in the extraction part 4 (see Fig. 4), the ultraviolet light having a wavelength of 240 nm or more and 280 nm or less is substantially prevented from being transmitted. This reliably suppresses the ultraviolet light in the wavelength band that possibly affect the human body from leaking to the outside of the housing 2, and the safety of the irradiation device with respect to the human body is further improved.

From a viewpoint of improvements in the safety to humans or animals, it is preferable that the ultraviolet light emitted from a light source unit be within a wavelength range of 190 nm or more and 237 nm or less, more preferably within a wavelength range of 190 nm or more and 235 nm or less, and particularly preferably within a wavelength range of 190 nm or more and 230 nm or less.

Furthermore, the ultraviolet light having a wavelength of less than 190 nm is absorbed into oxygen in the air, and this results in generation of ozone. In order to more effectively suppress the generation of ozone, it is desirable to use the ultraviolet light having a peak wavelength of 190 nm or more, more preferably 200 nm or more. For example, it is preferable that a peak wavelength of the ultraviolet light emitted from the light source unit be within a wavelength range of 200 nm or more and 237 nm or less, more preferably within a wavelength range of 200 nm or more and 235 nm and less, and further preferably within a wavelength range of 200 nm or more and 230 nm or less.

In the present description, the phrase "does not substantially transmit ultraviolet light" means that the ultraviolet light in a main light beam direction is suppressed to an ultraviolet light intensity of at least 5% or less with respect to an ultraviolet light intensity of a peak wavelength in a specific wavelength band. In the present invention, by using the optical filter 6, the intensity of the ultraviolet light of 240 nm or more and 300 nm or less is shielded to 5% or less with respect to the intensity of the peak wavelength. Note that, regarding the light in a wavelength band desired to be shielded by the optical filter 6, the intensity of the ultraviolet light transmitted through the optical filter 6 is preferably suppressed to 2% or less of the intensity of the peak wavelength. It is more preferable that the intensity of the ultraviolet light transmitted through the optical filter 6 be suppressed to 1% or less with respect to the intensity of the peak wavelength.

The optical filter 6 may have any form as long as the optical filter functions as a band pass filter that does not transmit the ultraviolet light in a specific wavelength band, and the arrangement place and form are not limited. As shown in Fig. 4, in addition to the optical filter 6 being arranged separated from the light source, the optical filter 6 may be configured so as to be in contact with the light source (as a specific example, the optical filter 6 may be laminated on a glass seal of the excimer lamp 3).

The optical filter 6 is formed by, for example, forming a dielectric multilayer film in which dielectric films having different refractive indexes are alternately laminated. Examples of the dielectric multilayer film include a dielectric multilayer film in which hafnium oxide (HfOz) layers and silicon dioxide (SiO₂) layers are alternately laminated, and a dielectric multilayer film in which SiO₂ layers and aluminum oxide (Al₂O₃) layers are alternately laminated. The dielectric multilayer film in which the HfOz layers and the SiO₂ layers are alternately laminated can reduce the number of layers for obtaining the same wavelength-selective characteristics as compared to the dielectric multilayer film in which the SiO₂ layers and the Al₂O₃ layers are alternately laminated, and thus can increase the transmittance of the selected ultraviolet light.

### [Transmittance change depending on incident angle to optical filter]

As described above, the optical filter 6 is constituted of the plurality of dielectric multilayer films having different refractive indices. However, the optical filter 6 constituted of the dielectric multilayer film has the transmittance inevitably changed depending on the incident angle of the ultraviolet light.

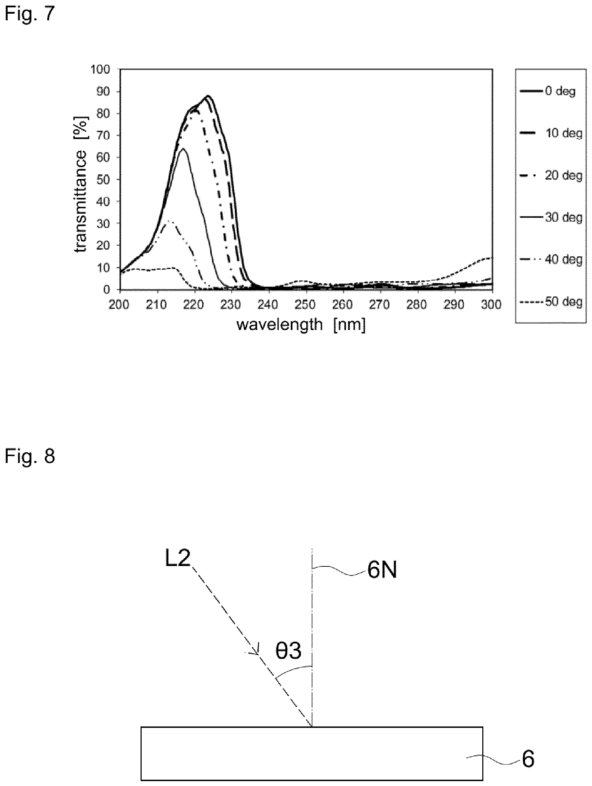

Fig. 7 is a graph illustrating an example of a transmission spectrum of the optical filter 6 for each incident angle when the ultraviolet light is incident on the optical filter 6. In the example in the graph, the optical filter 6 is designed assuming a case where the light-emitting gas of the excimer lamp 3 contains KrCl, that is, a case where the excimer lamp 3 emits the ultraviolet light whose main peak wavelength is 222 nm. Each curve in the graph is obtained by plotting a ratio of the intensity of light incident on the optical filter 6 to the intensity of light emitted from the optical filter 6 for each of the different wavelengths. As illustrated in Fig. 8, an incident angle is defined by an angle θ3 between a normal line 6N to the incident surface of the optical filter 6 and ultraviolet light L2 incident on the incident surface of the optical filter 6.

From the graph in Fig. 7, it can be seen that the optical filter 6 easily transmits a light component having a small incident angle (angle θ3) but does not easily transmit a light component having a large incident angle. The light component having a large incident angle does not enter the optical filter 6 and is reflected. As a result, the light emitted from the optical filter 6 has a higher ratio of a light component having a smaller incident angle than that of the light incident on the optical filter 6, and has a smaller light distribution angle. In other words, the optical filter 6 reduces the light distribution angle.

Under such circumstances, the above-described diverging optical system 5 can obtain a particularly remarkable effect in a case where the optical filter 6 that reduces the light distribution angle is used. Furthermore, even in a case where the light distribution angle is reduced by using the optical filter 6, the ultraviolet irradiation device 10 can obtain a large light distribution angle by disposing the diverging optical system 5 on the emission side of the optical filter 6.

A separation distance between the optical filter 6 and the diverging optical system 5 may be 0 mm or more and 3 mm or less. By setting the separation distance within the above range, the emission light of the optical filter 6 can be effectively incident on the diverging optical system 5, and the emission efficiency can be enhanced. The separation distance is represented by the shortest distance between the optical filter 6 and the diverging optical system 5. In the ultraviolet irradiation device 10 illustrated in Fig. 4, because the optical filter 6 is disposed so as to be in contact with a portion other than the concave surface of the diverging optical system 5, the separation distance is 0 mm.

### [Electrode block]

A surface 9r (see Fig. 3) of the electrode blocks (9a, 9b) facing the excimer lamp 3 function as a reflection surface of the ultraviolet light. In the present embodiment, the surface 9r is constituted of a plane inclined with respect to the surface of the extraction part 4. With this configuration, the direction of light traveling from the excimer lamp 3 to the electrode blocks (9a, 9b) can be changed to cause the light to travel to the extraction part 4. This can increase the emission efficiency of light.

The surface 9r may be a curved surface. In particular, by making the surface 9r a parabolic curved surface, the divergence angle from the excimer lamp 3 can be further directed, and the emission efficiency can be enhanced.

Al, an Al alloy, or stainless steel may be used for the electrode blocks (9a, 9b). These materials are conductive and have high reflectance of ultraviolet light.

In the present embodiment, it has been described that a part of the surface constituting the electrode blocks (9a, 9b) having a power feeding function functions as a reflection surface. However, a material having a reflection function different from that of the electrode blocks (9a, 9b) may be disposed on the surface of the electrode blocks (9a, 9b).

### [First modification]

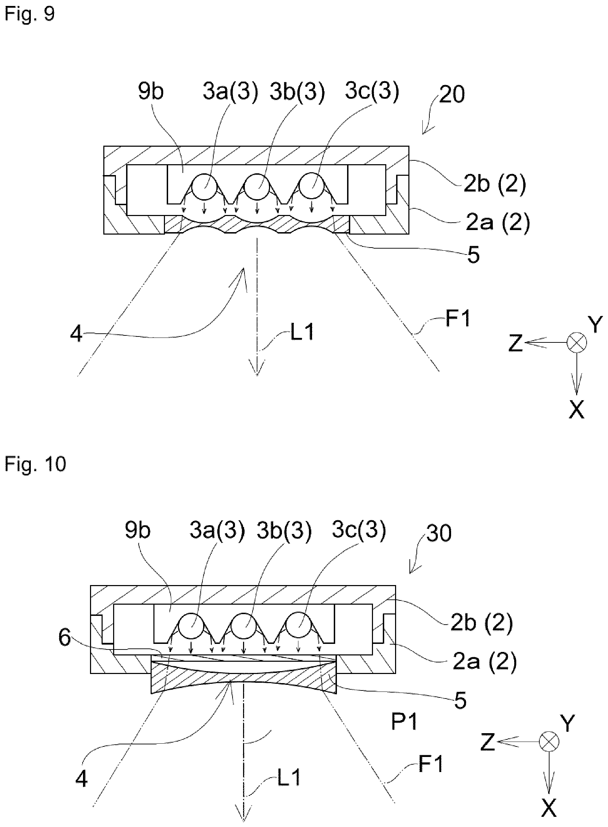

A first modification of the ultraviolet irradiation device is described with reference to Fig. 9. An ultraviolet irradiation device 20 does not include an optical filter that functions as a wavelength selection filter. In a case where a light source itself does not emit ultraviolet light that may affect the human body or in a case where the light source itself includes an optical filter, as in the first modification, the ultraviolet irradiation device 20 may not include the optical filter.

### [Second modification]

A second modification of the ultraviolet irradiation device is described with reference to Fig. 10. In an ultraviolet irradiation device 30, the diverging optical system 5 does not include the lens array in which the plurality of small lenses is aligned, but includes a single lens. Further, in this modification, a biconcave lens having a cylindrical surface (surface curved in a columnar shape) is illustrated, but lenses having other shapes such as a plano-concave lens and a concave meniscus lens may be used as long as the lens has a diverging function.

### [Method for using]

As a mode of using the ultraviolet irradiation device according to the present invention, the ultraviolet irradiation device can be disposed so that the emitted ultraviolet light is emitted toward a manned space to allow the ultraviolet irradiation device to emit the ultraviolet light. The manned space means a space that a person can enter regardless of whether or not a person is actually present. The manned space includes, for example, a space in a building such as a house, an office, a school, a hospital, or a theater, or a space in a vehicle such as an automobile, a bus, a train, or an airplane. The ultraviolet irradiation device 10 is disposed on a ceiling, a wall, a column, a floor, or the like facing the manned space so that the extraction part 4 faces the manned space. Then, the ultraviolet irradiation device 10 is turned on to emit the ultraviolet light to the manned space.

In this method for using, as in the related art, it is not necessary to emit the ultraviolet light while avoiding the human body, and it is possible to emit the ultraviolet light without unevenness (with small unevenness) to the entire manned space including the surface (skin or the like) of the human body, the surface of an object with which a human frequently comes in contact, or a space near the human body, which is the essential place where microorganisms should be most inactivated. Therefore, inactivation of microorganisms can be effectively performed.

The ultraviolet irradiation device may be incorporated in a lighting facility such as a fluorescent lamp or a light-emitting diode (LED). In the case where the ultraviolet irradiation device is incorporated in the lighting facility, the diverging optical system 5 used in the ultraviolet irradiation device may be shared with the diverging optical system for visible light used in the lighting facility.

### <Second embodiment>

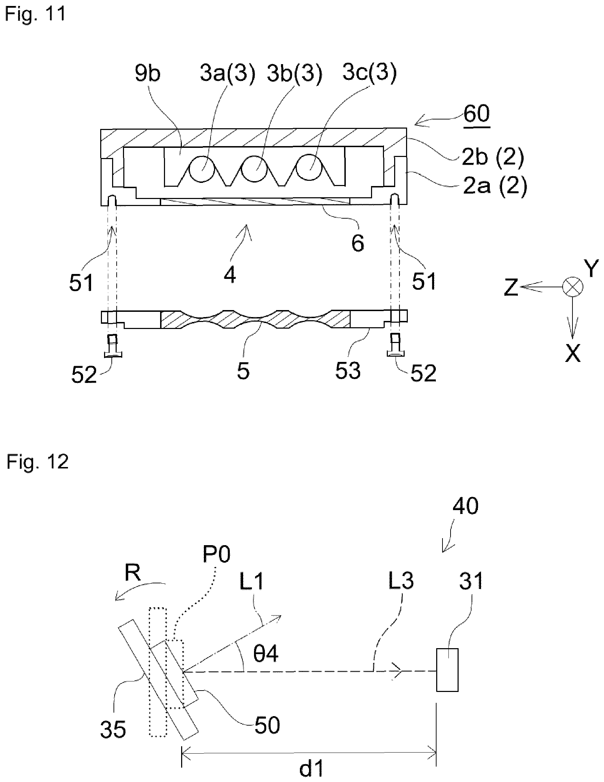

An outline of a second embodiment of an ultraviolet irradiation device is described with reference to Fig. 11. The second embodiment can be implemented in the similar manner as the first embodiment except for the following points. An ultraviolet irradiation device 60 of the second embodiment includes excimer lamps 3 (3a, 3b, 3c) that emit ultraviolet light of 190 nm to 240 nm, a housing 2 that accommodates the excimer lamps 3, and an extraction part 4 that extracts the ultraviolet light emitted from the excimer lamp 3 to the outside of the housing 2. Further, the ultraviolet irradiation device 60 includes, in the extraction part 4, an optical filter 6 that transmits the ultraviolet light in a wavelength band of 190 nm or more and 240 nm or less and does not substantially transmit the ultraviolet light in a wavelength band of 240 nm or more and 280 nm or less. However, as described in the first modification of the first embodiment, the optical filter 6 is not an essential component for the ultraviolet irradiation device 60.

The ultraviolet irradiation device 60 of the present embodiment does not include a diverging optical system 5. Instead, the housing 2 has attachment parts 51 for attaching the diverging optical system 5 so that the diverging optical system 5 can be retrofitted to the ultraviolet irradiation device 60. In the present embodiment, each of the attachment parts 51 is a screw hole. By fitting a screw 52 into the screw hole in a state where a holding frame 53 of the diverging optical system 5 is sandwiched, the diverging optical system 5 is attached to the ultraviolet irradiation device 60. The mode of the attachment part 51 of the diverging optical system 5 is an example, and various modes such as a hook and a hook-and-loop fastener can be applied. By retrofitting the diverging optical system 5 in this manner, the diverging optical system can be replaced with a desired one.

Although the embodiments of the ultraviolet irradiation device and the method for using the ultraviolet irradiation device have been described above, the present invention is not limited to the above-described embodiments at all, and various modifications or improvements can be made to the above-described embodiments without departing from the gist of the present invention.

For example, an example in which the excimer lamp 3 is used as the light source has been described, but a solid-state light source including a laser diode (LD) or an LED may be used as the light source.

For example, as the optical filter, an optical filter that transmits ultraviolet light in a wavelength band of 200 nm to 230 nm and does not substantially transmit a wavelength band of less than 200 nm and 230 nm to 280 nm may be used. By adding a wavelength band of less than 200 nm to the ultraviolet light that is not substantially transmitted, generation of ozone is suppressed, and the safety for the human body is further enhanced.

### [Examples]

For an example of the ultraviolet irradiation device illustrated in the above embodiments, the effect of increasing the light distribution angle by using the diverging optical system 5 has been confirmed. Fig. 12 is a schematic view of a measurement facility 40 for measuring the light distribution angle of the ultraviolet irradiation device. The measurement facility 40 includes an ultraviolet irradiation device 50, a rotary stage 35 on which the ultraviolet irradiation device 10 is placed, and an irradiance meter 31. Note that UV POWER METOR C8026 manufactured by Hamamatsu Photonics K.K. is used as the irradiance meter 31.

The measurement facility 40 includes the rotary stage 35, the ultraviolet irradiation device 50 placed on the rotary stage 35, and the irradiance meter 31 disposed at a position separated from the ultraviolet irradiation device 50 by a distance d1: 300 (mm). The position of the rotary stage 35 when the ultraviolet irradiation device 10 is arranged to face the irradiance meter 31 so that the irradiance meter 31 is positioned on the optical axis L1 of the ultraviolet irradiation device 50 is defined as an initial position P0. The rotary stage 35 is rotated from the initial position P0 in a rotation direction R illustrated in Fig. 12. In Fig. 12, the rotary stage 35 and the ultraviolet irradiation device 50 at the initial position P0 are indicated by dotted lines, and the rotary stage 35 and the ultraviolet irradiation device 50 after being rotated in the rotation direction R for a predetermined time are indicated by solid lines.

An angle θ4 formed between the optical axis L1 of the ultraviolet irradiation device 50 and the light beam L3 incident on the irradiance meter 31 from the ultraviolet irradiation device 50 represents a rotation angle. The irradiance was measured with the irradiance meter 31 while the ultraviolet irradiation device was emitted from the ultraviolet irradiation device 50, and meanwhile, the rotation angle θ4 was increased from the initial position P0 at which the rotation angle θ4 was 0 degrees (deg) (the rotary stage 35 was rotated) until the rotation angle θ4 reached 80 degrees (deg).

The ultraviolet irradiation device 50 has the same structure as the ultraviolet irradiation device 10 illustrated in Fig. 4. However, in the prepared ultraviolet irradiation device, four KrCI excimer lamps 3 are arranged in the Z direction. Three (S1, S2, S3) of the ultraviolet irradiation devices 50 were prepared. However, the diverging optical systems 5 of the three ultraviolet irradiation devices (S1, S2, S3) each satisfy the following conditions.

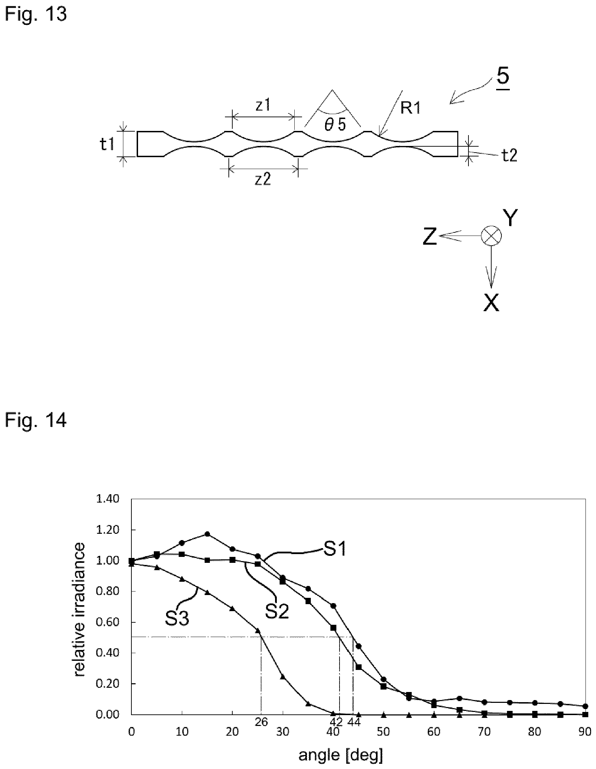

As illustrated in Fig. 13, the diverging optical system 5 of the ultraviolet irradiation device S1 is configured by arranging four small lenses (biconcave lenses having a cylindrical surface) in the Z direction. The diverging optical system 5 has the following dimensions.
Lens thickness t1: 8 mm
Lens depth t2: 3.34 mm
Radius of curvature R1 of concave surface of lens: 8 mm
Angle θ5 of concave surface of lens: 108 degrees [deg.]
Lens width z1 in z direction: 13 mm
Lens pitch z2 in z direction: 14 mm

As illustrated in Fig. 13, the diverging optical system 5 of the ultraviolet irradiation device S2 is configured by arranging four small lenses (biconcave lenses of a cylindrical type) in the Z direction. The diverging optical system 5 has the following dimensions.
Lens thickness t1: 5 mm
Lens depth t2: 1.74 mm
Radius of curvature R1 of concave surface of lens: 13 mm
Angle θ5 of concave surface of lens: 60 degrees [deg.]
Lens width z1 in z direction: 13 mm
Lens pitch z2 in z direction: 14 mm

The optical lens of the lens array used in the diverging optical system 5 of the ultraviolet irradiation device S2 has a larger radius of curvature than the optical lens used in the diverging optical system 5 of the ultraviolet irradiation device S1. That is, a refractive power of the optical lens of the ultraviolet irradiation device S1 is larger than a refractive power of the optical lens of the ultraviolet irradiation device S2.

The ultraviolet irradiation device S3 is an ultraviolet irradiation device without the diverging optical system 5 and is illustrated as a comparative example.

For each of the ultraviolet irradiation devices (S1, S2, S3), the relative irradiance was obtained based on the measured irradiance results. The relative irradiance is obtained by dividing an irradiance measurement value at an arbitrary rotation angle by the irradiance measurement value at a rotation angle of 0 degrees (deg). That is, the relative irradiance is a relative value of the irradiance of the light beam traveling at an arbitrary angle in a case where the irradiance of the light beam traveling in the optical axis direction is 1. Fig. 14 is a graph illustrating the relative irradiance with respect to a change in rotation angle.

The ultraviolet irradiation devices (S1, S2) included in the diverging optical system 5 exhibit a higher relative irradiance with respect to a wide angle range than the ultraviolet irradiation device S3 without the diverging optical system 5. Regarding the rotation angle at which the relative irradiance is 0.50 (measured irradiance that is half the irradiance at the rotation angle of 0 degrees (on the optical axis)), the ultraviolet irradiation device S1 has an angle of about 44 degrees (deg), the ultraviolet irradiation device S2 has an angle of about 42 degrees, and the ultraviolet irradiation device S3 has an angle of only about 26 degrees. Considering that the light distribution angle is obtained at twice the rotation angle at which the relative irradiance is 0.50, it can be said that the light distribution angle is enlarged by about 36 degrees by providing the diverging optical system in the ultraviolet irradiation device S1, and the light distribution angle is enlarged by about 32 degrees by providing the diverging optical system in the ultraviolet irradiation device S2.

In the above, the example in the presence or absence of the diverging optical system of the lens array has been described, but it is presumed that the same tendency is exhibited also in the ultraviolet irradiation device not including the optical filter or the ultraviolet irradiation device including the diverging optical system including the single lens.

Next, the ultraviolet irradiation device S1 is compared with the ultraviolet irradiation device S2. In the case of the ultraviolet irradiation device S1 including the optical lens having a large refractive power, the relative irradiance of the light beam at an angle of about 15 degrees from the optical axis exceeds the relative irradiance of the light beam on the optical axis by about 0.2 points. On the other hand, in the case of the ultraviolet irradiation device S2 including the optical lens having a small refractive power, there is no light beam in which the relative irradiance of the light beam exceeds 1.1. That is, the ultraviolet irradiation device S2 has a more uniform orientation distribution than the ultraviolet irradiation device S1. Therefore, as described at the beginning, the ultraviolet irradiation device S2 having a more uniform alignment distribution can ensure the safety at a higher level than the ultraviolet irradiation device S1, and is less likely to be restricted by the upper limit of the ultraviolet light emission amount.

By setting the optical lens to have the refractive power within a certain numerical range, the ultraviolet irradiation device can be efficiently used. In the case of the lens array, the radius of curvature is preferably 10 mm or more, and more preferably 13 mm or more from the above-described experimental results. In addition, in order to obtain a sufficient divergence effect, the radius of curvature is more preferably equal to or less than the pitch at which the light sources are arranged.

### DESCRIPTION OF REFERENCE SIGNS

- 2: Housing
- 2a, 2b: First frame
- 3: Excimer lamp
- 4: Extraction part
- 5: Diverging optical system
- 5a: Small lens
- 6: Optical filter
- 7a, 7b: Power feeding line
- 8a, 8b: Connection terminal
- 9a, 9b: Electrode block
- 9r: Surface (to be reflective surface of electrode block)
- 10, 20, 30, 50, 60: Ultraviolet irradiation device
- 31: Irradiance meter
- 35: Rotary stage
- 40: Measurement facility
- 51: Attachment part
- 52: Screw
- 53: Holding frame

## Claims

1. An ultraviolet irradiation device comprising:
a light source that emits ultraviolet light having a main emission wavelength belonging to a range from 190 nm to 240 nm;
a housing that accommodates the light source;
an extraction part that extracts the ultraviolet light emitted from the light source to an outside of the housing; and
a diverging optical system that enlarges a light distribution angle of the ultraviolet light passing through the extraction part.

2. The ultraviolet irradiation device according to claim 1, further comprising
an optical filter provided on an incident side of the diverging optical system, the optical filter transmitting the ultraviolet light having the main emission wavelength belonging to a wavelength band from 190 nm to 240 nm and not substantially transmitting the ultraviolet light in a wavelength band of 240 nm to 280 nm.

3. The ultraviolet irradiation device according to claim 2, wherein a separation distance between the optical filter and the diverging optical system is 0 mm or more and 3 mm or less.

4. The ultraviolet irradiation device according to any one of claims 1 to 3, wherein the diverging optical system includes a lens array.

5. The ultraviolet irradiation device according to claim 4, wherein
the light source includes at least one excimer lamp,
the lens array includes small lenses whose number is equal to a number of the excimer lamps, and
the small lenses each have a longitudinal direction that extends along a longitudinal direction of the excimer lamp.

6. A method for using an ultraviolet irradiation device comprising
disposing the ultraviolet irradiation device according to any one of claims 1 to 3 such that at least a part of the ultraviolet light to be emitted is emitted toward a space, and causing the ultraviolet irradiation device to emit the ultraviolet light.

7. An ultraviolet irradiation device comprising:
a light source that emits ultraviolet light having a main emission wavelength belonging to a range from 190 nm to 240 nm;
a housing that accommodates the light source; and
an extraction part that extracts the ultraviolet light emitted from the light source to an outside of the housing, wherein
the housing includes an attachment part configured to attach a diverging optical system to the housing, the diverging optical system enlarging a light distribution angle of the ultraviolet light passing through the extraction part.

8. An ultraviolet irradiation method comprising:
emitting ultraviolet light having a main emission wavelength belonging to a range from 190 nm to 240 nm from a light source; and
enlarging a light distribution angle of the ultraviolet light using a diverging optical system.
